# EUROPEAN PATENT APPLICATION

(11) **EP 4 620 476 A1**
(43) Date of publication of application: **24.09.2025**
(21) Application number: 24726516.8
(22) Date of filing: 09.01.2024
(51) Int. Cl.: A61K 31/724, A61K 36/00, A61K 31/155, A61L 101/50, A61L 2/16, A61L 12/08, A61K 31/65, A61K 31/19

(54) **ANTIVIRAL COMPOSITION CONTAINING INCLUSION COMPOUNDS WITH CYCLODEXTRINS AS ACTIVITY MODULATORS AND ANTISEPTIC ACTIVES AND USES THEREOF**

(30) Priority: 17.11.2022 BR 102022023350
(71) Applicant: Silva, Renata Moisés Iwamizu, 13254-432 Itatiba (BR)
(72) Inventor: Silva, Renata Moisés Iwamizu, 13254-432 Itatiba (BR)
(74) Representative: Patentree
(86) International application number: PCT/IB2024/050204
(87) International publication number: WO 2024/105648

(57) **Abstract**

The present invention relates to compositions comprising inclusion compounds with cyclodextrins and antiseptic actives for the elimination of viruses from the orders *Herpesvirales, Caudovirales, Mononegavirales, Nidovirales, Picornavirales, Tymovirales,* and from the family *Retroviridae.* More specifically, the present invention discloses a method for modulating the activity of an antiseptic agent for virucidal action through its association with beta-cyclodextrin and/or its derivatives, in a combined action that exhibits high effectiveness in eliminating viruses-not merely weakening them-while using low concentrations of antiseptic actives and exhibiting low toxicity

## Description

The present invention relates to compositions comprising inclusion compounds with cyclodextrins and antiseptic actives for the elimination of viruses from the orders *Herpesvirales, Caudovirales, Mononegavirales, Nidovirales, Picornavirales, Tymovirales,* and from the family *Retroviridae.* More specifically, the present invention is directed to compositions with antiviral activity, having cyclodextrins in association with an antiseptic active as the main agents, in a combined action that demonstrates high efficacy in eliminating the virus-not merely weakening it-while using low concentrations of encapsulated antiseptic actives and exhibiting low toxicity.

Awareness of the risks that viruses pose to humanity has been increasing. Among the widely known virus orders is *Herpesvirales.* On average, 80% of the global adult population carries the *Herpes simplex* virus, with most individuals being asymptomatic or presenting mild symptoms. The disease typically manifests as a burning sensation, itching, stinging, or general discomfort in the area of the skin where the virus becomes active. Subsequently, the affected area becomes reddened, followed quickly by the formation of blisters or vesicles. After a few days, these blisters rupture and release viral fluid, marking a period of high transmissibility. Scabs (crusts) then form over the damaged area, corresponding to tissue healing and the completion of the *Herpes simplex* virus cycle.

During the first outbreak of the *Herpes simplex* virus, the individual may experience fever and general malaise. The duration of the illness ranges from a few days to several weeks. However, despite the end of the infectious activity, the virus remains in a latent replication state, residing in the trigeminal and/or sacral nerves for the lifetime of the host.

Although the disease is associated with mild symptoms, considering that it manifests on the host's skin-such as in the lip region in the case of *Herpes simplex virus* type 1-there is a significant psychological impact due to the presence of blisters, sores, and the visible nature of this contagious condition in social settings. Therefore, reducing the duration of the *Herpes* virus cycle helps minimize its impact on the host, both physically and psychologically.

Despite the pain, itching, burning sensation, and the care required during an active herpes outbreak on the skin, the most detrimental aspect is often the decline in the patient's self-esteem due to how others perceive the condition during its latent phase. Although the vast majority of the global population carries the *Herpes* virus in a dormant state-often without any active symptoms-social stigma remains the most significant challenge associated with the disease.

Oral herpes leads to social withdrawal, as its presence is visibly apparent. There are reports of patients describing themselves as "unclean and uncomfortable" due to the curious looks they receive. Genital herpes, caused by *Herpes simplex virus* type 2, tends to be more severe; the lesions are typically larger, more painful, and highly disruptive, as outbreaks can recur frequently in short intervals due to the high local humidity. Genital herpes restricts sexual relationships and causes emotional distress for both the affected individual and their partners.

*Herpes zoster,* commonly known as shingles or *zona,* is an infectious disease caused by the *Varicella-Zoster* virus, which belongs to the same *Herpesviridae* family as the *Herpes simplex* virus. Initial exposure to the virus typically occurs during childhood, resulting in chickenpox. After the acute phase of infection, the immune system generates antibodies, controls viral replication, and the disease resolves spontaneously. However, the virus remains latent in the nerve endings of the skin and migrates to certain ganglionic chains located near the spinal cord and brain, where it can persist for extended periods, awaiting an opportunity-such as immunosuppression-to reactivate and multiply. Upon reactivation in adulthood, *Herpes zoster* causes a dermatological outbreak characterized by multiple reddish vesicles that typically last 3 to 5 days, often resolving within 7 to 10 days as the blisters dry out and begin forming crusts. The associated pain, however, may persist for up to a month before complete resolution.

*Herpes zoster* becomes more sensitive in individuals over the age of 50, especially when they experience physical or psychological stress, sleep deprivation, diabetes mellitus, cancer, the use of immunosuppressive drugs, or are HIV-positive. Oral antiviral treatment is initiated within the first 72 hours and aims to reduce the viral cycle and the severity of the vesicles. *Acyclovir, Valacyclovir,* and *Famciclovir* are the commonly used medications. However, the dosages of these drugs are high during treatment, with *Valacyclovir* prescribed at 1000 mg three times a day, *Famciclovir* at 500 mg three times a day, and *Acyclovir* at 800 mg five times a day.

There are no reports in the prior art or state of the art of pharmaceutical compositions that result in an effective treatment against *Herpes zoster or Herpes simplex* viruses. The main active agents currently available merely reduce the viral cycle or alleviate symptoms, particularly when the patient's immune system is compromised. Furthermore, the use of high concentrations of these compounds may lead to adverse side effects in the body.

The treatment options currently available on the market for *Herpes simplex, Herpes zoster,* and *Varicella zoster* viruses are capable of minimizing the size of the lesions; however, the host still undergoes all stages and discomfort associated with the disease. Various formulation matrices-such as ointments, liquid dressings, patches, and gels-are disclosed in the prior art for direct application to the affected area, as well as tablets or capsules for oral administration and injectable solutions. The active pharmaceutical ingredients in these materials include compounds already described in the state of the art, such as *acyclovir* (Zovirax), *silver sulfadiazine* combined with *cerium nitrate* (Dermacerius HS gel), or *penciclovir* (Penvir Labia)-all of which act against the virus but do not terminate the viral cycle. Instead, they primarily reduce secondary symptoms such as itching, burning, and blister formation.

Drugs used against herpes viruses are administered intravenously, orally, or topically. The concentration of the active ingredient depends on the type of herpes virus, the age and weight of the infected individual, and the duration of treatment. The low bioavailability of commercially available drugs poses a challenge to their effectiveness against the virus. *Acyclovir,* for example, has an oral bioavailability of only 15% to 20%, with plasma protein binding of less than 20%. This results in reduced therapeutic efficacy and necessitates higher dosage levels, which, in turn, may increase the likelihood of adverse side effects in the patient's body.

In addition to the limited effectiveness of commercially available drugs in reducing the viral cycle, there is also the occurrence of potential side effects. For example, *Acyclovir* must be administered at reduced doses in patients with renal insufficiency, particularly in the elderly and those with impaired kidney function. Its intravenous administration should be performed with caution in patients with underlying neurological disorders, significant hypoxia, or severe hepatic or electrolyte abnormalities. *Penciclovir,* on the other hand, is associated with a range of adverse effects, including drowsiness, dysmenorrhea, erythema multiforme, hallucinations, hyperbilirubinemia, cognitive dysfunction, jaundice, leukopenia, migraine, neutropenic disorders, Stevens-Johnson syndrome, thrombocytopenic syndrome, toxic epidermal necrolysis, urticaria, and vomiting.

Cyclodextrins (CDs) are widely used due to their high commercial availability, relatively low cost, low toxicity, established use in the formulation of inclusion compounds, and their role as excipients in drug delivery vehicles and as anti-aggregation agents. New formulations involving cyclodextrins continue to be reported, generating renewed scientific interest, particularly in the fields of medicine and biomedicine.

For many years, cyclodextrins were considered solely as excipients. However, more recent research has demonstrated that these oligosaccharides can no longer be regarded merely as excipients in pharmaceutical formulations. Serendipitously, it was discovered that hydroxypropyl-beta-cyclodextrin aids in slowing the progression of Niemann-Pick disease type C. Based on studies showing similar outcomes, cyclodextrins have recently been classified as active pharmaceutical ingredients (APIs) by both the Food and Drug Administration (FDA) and the European Medicines Agency (EMA).

The compounds formed as a result of the interaction between the cyclodextrin cavity and various molecules have been termed "host-guest interactions," and subsequently referred to as "inclusion compounds." Typically, the inclusion compound is formed through weak interactions within the internal cavity of the host, rendering the host-guest interaction reversible. The formation of inclusion compounds results from a dynamic equilibrium of association and dissociation between the free guest and the free host. This process is governed by the formation constant (Kf). The stability constant (Ka) of the inclusion compound is determined by the equilibrium between the associated and dissociated forms in aqueous media. The association and dissociation of the inclusion compound formed by cyclodextrin (CD) molecules and the guest are governed by a thermodynamic equilibrium. The preferred position of the guest within the cavity depends on steric interactions and functional groups of the guest molecule's structure, the medium in which the inclusion compound is present, the physical conditions of the environment, and the constants mentioned above.

The properties of cyclodextrins are fully utilized in the present invention. Their ability to establish specific molecular encapsulation interactions, through the formation of non-covalent bonds such as hydrophobic interactions, van der Waals forces, and hydrogen bonds, is crucial for the success of the technology. Since these are weak bonds in the inclusion compounds, the strategy of encapsulation at an initial stage and the release of the guest at a later stage create a sequence of activities that are essential for the effectiveness of the present invention.

Due to the toroidal structure of cyclodextrins, with their hydrophobic cavity and more hydrophilic external surface, a favorable interaction occurs between the compound to be encapsulated and the cyclodextrin cavity. The ideal approach in the present invention is to use active ingredients where the interaction between the active ingredient and the cyclodextrin cavity occurs, but is weaker than the interaction that will naturally occur between the microorganism structure and the cavity.

Studies such as document US20050014719, from 2014, demonstrate the development of techniques that prove the ability of cyclodextrins to interact with the viral structure and weaken it. The work of Wudiri and Nicola, from 2017, details the form of interaction specifically related to the cholesterol in the viral structure, confirming that cholesterol is relevant in the virus's fusion step with the cell membrane and, as a result, decreases the virus's effectiveness, at least temporarily. The article also states that cyclodextrin is not capable of eliminating the virus, thus not preventing it from infecting the cell via the endocytosis process. (Wudiri GA, Schneider SM and Nicola AV (2017) Herpes Simplex Virus 1 Envelope Cholesterol Facilitates Membrane Fusion. Front. Microbiol. 8:2383).

The article titled "Virucidal efficacy of chlorhexidine: a systematic review," from 2022, presents a review of the virucidal efficacy of chlorhexidine (CHX) compared to other substances used in the oral cavity. Studies were conducted against Herpes Simplex Virus Type-1 (HSV-1), Influenza A (InfluA), and human coronavirus (HCoV), comparing the virucidal efficacy of CHX with essential oils, quaternary ammonium compounds, iodine povidone, hydrogen peroxide, a negative control substance, and no therapy. Randomized clinical trials demonstrated the virucidal activity of CHX against HSV-1, InfluA, and HCoV. In the study, CHX is present in its free form in the biological medium, with no investigation of its application in conjunction with or encapsulated in cyclodextrin, or its structural variations, or in any type of inclusion compound. However, even though CHX showed activity against the herpes virus, the toxicity of its free concentration used in the test was not evaluated for human cells.

It is already reported in the prior art and state of the art that the incorporation of chlorhexidine and its salts into cyclodextrins alters their physicochemical characteristics, such as their efficacy against pathogenic microorganisms. This formation of inclusion compounds, using varying molar ratios, results in a controlled release of chlorhexidine, which produces a prolonged action effect of this product in an application matrix. Additionally, due to the slow release, the free concentration of chlorhexidine is drastically reduced, and consequently, the toxicity of this active ingredient to healthy human cells is minimized.

Document RU 2535052, from 2014, refers to medicines, namely a pharmaceutical composition containing derivatives of lysine, proline, and triterpenic acid, which can be used in medicine for the treatment and prevention of viral infections caused by RNA and DNA viruses, such as influenza, herpes, herpes zoster, lichen, human papillomavirus, adenovirus, as well as infections caused by Gram-positive and Gram-negative bacteria, without including any inclusion compound, only free chlorhexidine.

Document US20120321603, from 2011, titled "Composition and method for stabilization and delivery of therapeutic molecules," presents a composition and method for the treatment of alpha, beta, and gamma-cyclodextrin and a natural molecule or its fragment, where the natural molecule is glutathione, non-acetylated, non-esterified, and not bound to fatty acid, with the composition being administered parenterally and not interventively.

Document US6060597, from 1997, refers to a cyclic oligosaccharide or its salts comprising glucose molecules cyclically linked via α-1,6 bonds and having at least one S-oxo acid group attached, and an agent for preventing or treating retroviral diseases, which includes said cyclic oligosaccharide or its salts as an active ingredient. The new sulfated cyclic isomaltoligosaccharide inhibits the infection of host cells by various retroviruses, such as the AIDS virus, etc., so effectively that it has a significant preventive and therapeutic effect on diseases caused by retroviruses. Consequently, the said oligosaccharide can be used as an agent in the prevention or treatment of retroviral diseases in the pharmaceutical industry. The patent mentions the group of sulfated cyclic oligosaccharides, which is not used in the technology for which protection is being sought.

Document WO 1990000596, from 1990, reveals new methods for inhibiting the infection of cells by a virus. Carbohydrate blocking agents are provided, capable of interacting with cells or viruses that are brought into contact with the cells under selected conditions, to effect the interaction of the carbohydrate blocking agent with the cells or viruses, resulting in interference with the virus's binding to the cells. The composition described does not mention the use of inclusion compounds or the combination of a cyclic oligosaccharide with an antiseptic agent for the elimination of the herpes virus.

The present invention takes into account prior knowledge and finds a way not only to weaken the virus but also to eliminate it, indicating the modulation of activity through the formation of inclusion compounds associated with antiseptic agents that, in their free form, do not show efficacy against certain virus families at concentrations safe for mammalian cells. However, after the formation of the inclusion compound with cyclodextrins, they can exhibit high efficacy. By forming an inclusion compound with cyclodextrin and an antiseptic agent, the present invention demonstrates high effectiveness in eliminating the virus, not just weakening it. The modulation of activity is associated with the interaction of the cyclodextrin with the external structure of the virus, actively participating in its elimination process, and in collaboration with the antiseptic agent encapsulated in the previously formed inclusion compound, it eliminates the pathogenic microorganism.

Thus, we request protection for the use of the cyclodextrin inclusion complex with antiseptic agents to modulate the activity of virus elimination, especially those from the herpesvirales, caudovirales, mononegavirales, nidovirales, picorvirales, and tymovirales orders, and the retroviridae family.

### DETAILED DESCRIPTION OF THE TECHNOLOGY

The present invention relates to compositions containing inclusion compounds with cyclodextrins and antiseptic agents for the elimination of viruses from the orders *Herpesvirales, Caudovirales, Mononegavirales, Nidovirales, Picornavirales, Tymovirales,* and from the *Retroviridae* family. More specifically, the invention concerns compositions with antiviral activity, having cyclodextrins in association with antiseptic agents as the main components, in combined action methods that demonstrate high efficacy in virus elimination rather than merely weakening, using low concentrations of encapsulated antiseptic agents and exhibiting low toxicity.

The present invention introduces, as a novelty, the modulation of the activity of antiseptic agents that do not exhibit satisfactory antiviral activity, and which, after encapsulation with cyclodextrin at varying molar concentrations, become effective antiviral agents, applicable at low concentrations and safe for both human and veterinary use.

For example, chlorhexidine and its salts are not active against viruses from the *Herpesviridae* family at concentrations that are safe for mammalian cells. However, the combination of CHX with cyclodextrin, at varying molar concentrations, exhibits antiviral activity and enables the use of low and safe concentrations of CHX for this purpose.

The antiviral composition comprises encapsulating agents, either free or not, consisting of cyclic oligosaccharides, at least one antiseptic agent in an inclusion compound with a cyclic oligosaccharide, and excipients or a mixture thereof.

The encapsulating agents are, in particular, beta-cyclodextrin and/or its structural derivatives, or a mixture thereof. The structural derivatives of beta-cyclodextrin include hydroxypropyl-beta-cyclodextrin, sulfobutylether-beta-cyclodextrin sodium (betadex sulfobutyl ether sodium), hydroxymethyl-beta-cyclodextrin, methyl-beta-cyclodextrin, 2,6-dimethyl-beta-cyclodextrin, and hydroxyethyl-beta-cyclodextrin.

The antiseptic agent should be a cationic, anionic, zwitterionic, or neutral antimicrobial agent, such as those from the bisbiguanide class, including chlorhexidine or its acetate, gluconate, or digluconate salts, or from the tetracycline class, such as doxycycline, eravacycline, minocycline, omadacycline, or a combination thereof.

The antiseptic agent should also be selected from groups comprising classes of saturated or unsaturated chain organic acids, with a main carbon chain containing between 2 and 8 carbon atoms, or combinations of these acids, in concentrations ranging from 0.01 to 30% w/v. It may also be selected from groups comprising classes of essential oils or hydrosols of essential oils derived from natural sources such as thyme, eucalyptus, oregano, basil, peppermint, rosemary, wild rosemary, pequi, geranium, citronella, palmarosa, Surinam cherry (pitanga), cypress, tea tree, ginger, lemongrass, lemon balm, or propolis, or combinations thereof, in concentrations ranging from 0.05 to 99% w/w.

The excipients should be selected from the group consisting of humectants, emulsifiers, preservatives, thickeners, sweeteners, stabilizers, colorants, antioxidants, surfactants, and/or flavoring agents, in concentrations ranging from 0.02 to 99% w/w.

The antiseptic agent and the encapsulating agent should be in molar ratios of 8:1 to 1:10, respectively, and comprise 0.01 to 20% w/w of the excess free encapsulating agent in the product and/or its structural variations or combinations thereof.

The technology presents the use of beta-cyclodextrin and/or its structural variations or combinations thereof as a modulator of antiviral activity of antiseptic agents that, when free, are ineffective against viruses but, through encapsulation with cyclodextrin or its structural variations, exhibit activity against viruses selected from the groups of the orders Herpesvirales, Caudovirales, Mononegavirales, Nidovirales, Picornavirales, Tymovirales, and the Retroviridae family, preferably against viruses from the Herpesviridae and Retroviridae families. This technology may have pharmaceutical applications, such as antibiotics, tablets, injectables, and related forms, as well as for veterinary use.

The antiviral composition can be used for the topical and systemic treatment of diseases caused by HSV-1, HSV-2, varicella-zoster (HHV-3), Epstein-Barr (HHV-4), cytomegalovirus (HHV-5), HHV-6, HHV-7, and Kaposi's sarcoma (HHV-8). For topical treatment, it should be selected from the group comprising epithelial tissues, skin, mouth, fingers, nails, hair, mammary glands, perineal region, genitalia, or mucous membranes.

They can be used in the production of surface disinfectant sanitizers, for the production of formulations for topical use or for treatment of the environment, surface sanitation, air sanitation, or in air purification equipment.

The composition can be used in aqueous solution, powder, paste, cream, wet gel, xerogels, aerosols, spray or foam, tablets, capsules, incorporated into cellulosic materials, polymers, or fabrics.

This technology can be better understood with the following examples, which are not limiting in nature.

### Example 1. Initial processes for preparing inclusion compound compositions in different molar proportions.

The preparation process of the solutions with the inclusion compounds was carried out by adding the compound to be encapsulated, such as chlorhexidine salt, into an aqueous solution containing beta-cyclodextrin encapsulant at 60°C, maintaining the molar ratios of 1:2 (active A2) or 1:3 (active A3), following the procedure reported in the literature with slight modifications (CORTÉS et al., The chlorhexidine:β-cyclodextrin Inclusion compounds: preparation, characterization and microbial evaluation. Journal of Inclusion Phenomena and Macrocyclic Chemistry, 40, 2001, 297-302).

### Example 2. Initial process of preparing the solution of active A2 in 99% (w/w) hydrosol.

The process of preparing the solution of active A2 in 99% (w/w) hydrosol was carried out by obtaining the isolated geranium hydrosol solution with an organic active concentration between 1% and 5% in solution. A specific amount of the solid active A2 was added to the geranium hydrosol solution to achieve a final concentration of 1% of this active.

The mixture was kept under agitation for a period of 10 minutes until the complete solubilization of active A2. After preparation, the final solution maintained the organoleptic properties of the selected hydrosol.

### Example 3. Evaluation of Cytotoxicity and Virucidal Activity of the Inclusion Compounds.

The product was evaluated for its effective concentration against the virus without causing toxicity to the tested cells. The virus (HSV-1) was added to different dilutions of the product and incubated for 1 hour at 37°C. Then, the solution was diluted (1:10) and added to Vero cells (ATCC CCL-81) for incubation for 72 hours at 37°C and 5% CO2. Positive control (virus without treatment with the inclusion compounds) and negative control (cells without virus and without treatment) were used, under the same conditions, in all tests. The results were evaluated by MTT (Mosmann, 1983; Sieuwerts et al., 1995). The assays were carried out in a NB-2 laboratory (Biosafety Level 2) following Good Laboratory Practices (GLP) and in accordance with the recommendations of ANVISA (Art. 1 and Art. 3 of IN 04/13 and IN 12/16).

The active A2 and active A3 eliminated 99.9% of HSV-1 particles at concentrations of 60 µg/mL. The inclusion compound in active A2 maintained this inhibition percentage up to 15 µg/mL (Table 1). The digluconate salt of chlorhexidine showed toxicity in Vero cells above 50% at 3 µg/mL (CC50 > 3 µg/mL), and at this concentration, it was unable to inactivate HSV-1.

**Table 1. Percentage of viral inactivation, based on the virucidal assay, of HSV-1 treatments with nanoparticles A2 and A3.**

| | **Viral inhibition (%)** | |
|---|---|---|
| **Concentrations (µg/mL)** | **active A2** | **active A3** |
| 60 | 99,9 | 99,9 |
| 30 | 29,2 | 99,9 |
| 15 | 18,4 | 99,9 |

### Example 4. Study of Antiviral Activity Against Herpes Virus.

Studies conducted at varying concentrations demonstrated antiviral activity against Herpes virus ranging from 99.9000% to 18.4000% (Table 2). Among the molar ratios tested, active compound A3 showed the highest activity, with antiviral efficacy exceeding 99% at a concentration of 15.00 µg mL⁻¹.

**Table 2. Antiviral activity data of molar variations of the inclusion compound against Herpes virus.**

| **Viral Envelope** | | **active A2 (µg/mL)** | | **active A3 (µg/mL)** |
|---|---|---|---|---|
| *Herpes virus* | 99,9000% | 60,00 | 99,9000% | 60,00 |
| *Herpes virus* | 29,2000% | 30,00 | 99,9000% | 30,00 |
| *Herpes virus* | 18,4000% | 15,00 | 99,9000% | 15,00 |

### Example 5. Study of antiviral activity against Respiratory Syncytial Virus (RSV), in vitro.

The studies against RSV virus were conducted with active ingredients A2 and A3. The cells used in the tests were human laryngeal carcinoma cells (ATCC CCL-23) (HEp-2), and the Respiratory Syncytial Virus (RSV, Long strain, single-stranded negative-sense RNA virus, enveloped).

The methodology used in the tests followed the described steps: initially, the cells were cultured in 96-well microplates with Dulbecco's Modified Eagle Medium (DMEM), supplemented with 10% fetal bovine serum, 100 µg/mL streptomycin, 100 IU/mL penicillin, and 2.5 µg/mL amphotericin B. The cells were maintained at 37°C and 5% CO2 for 24 hours until a monolayer formed (104 cells/mL). For the antiviral test, the products were added in contact with RSV (V/V) - approximately 250 infective doses for 50% of the cell cultures (ID50), in microtubes, at the recommended concentrations and times. The solutions (active ingredients + virus) were then serially diluted (base 10) and inoculated (8 repetitions each) into HEp-2 cells, followed by incubation for 72 hours at 37°C and 5% CO2.

The controls used, under the same conditions, in all tests: i) Viral Control (VC) - cells inoculated with virus and medium, in the absence of active ingredients; ii) Cell Control (CC) - cells in the absence of virus and active ingredients; and iii) Cytotoxicity - cells in the absence of virus and presence of the test concentrations of the active ingredients. The viral titers were determined using the calculation described by Reed & Münch (1938). The virucidal activity was determined by the reduction in viral titer compared to the VC and visualized by the reduction in the cytopathic effect (CPE).

Evaluation of the virucidal effect of the active ingredients at concentrations of 1.725% and 0.86% for active A2 and 2.25% and 1.125% for active A3 after contact for 1 and 15 minutes with the viruses: PV and RSV, in vitro. Evaluation of the virucidal effect of the active ingredients at concentrations of 1.725% and 0.686% for active A2 and 2.25% and 1.125% for active A3 after contact for 5, 30, and 60 minutes with HSV-1, in vitro.

The active ingredients did not inactivate PV at any of the concentrations and contact times evaluated. On the other hand, they inactivated RSV at the minimum contact time, down to the lowest concentrations for both active ingredients A2 and A3 (Table 3).

**Table 3. Viral inactivation percentage (%IV, from 0 to 99.9%) by virucidal effect for PV and RSV at different contact times and concentrations of active ingredients A2 and A3.**

| **Active A2** | **Active A3** | | | | | | |
|---|---|---|---|---|---|---|---|
| **Concentration (%)** | **Time** | **PV (% IV)** | **RSV (%IV)** | **Concentration (%)** | **Time** | **PV (%IV)** | **RSV (%IV)** |
| 2,25 | 1 min | 0 | 99,9 | 1,725 | 1 min | 0 | 99,9 |
| | 15 min | 0 | 99,9 | | 15 min | 0 | 99,9 |
| 1,125 | 1 min | 0 | 99,9 | 0,86 | 1 min | 0 | 99,9 |
| | 15 min | 0 | 99,9 | | 15 min | 0 | 99,9 |

When evaluated for HSV-1, active A2 inactivated the viral particle at the shortest contact time (5 minutes), down to the lowest concentration tested (0.686%). For active A3, 99.9% inactivation was achieved only at the highest concentration (2.25%), with the contact time with the viral particle increased to 60 minutes (Table 4).

**Table 4. Percentage of viral inactivation (%VI, from 0 to 99.9%) by virucidal effect for HSV-1, at different contact times and concentrations of active A2 and A3.**

| | **HSA-1** | | | |
|---|---|---|---|---|
| | **Active A2** | | **Active A3** | |
| **Time** | **Concentration (%)** | **% IV** | **Concentration (%)** | **%IV** |
| 5 min | 1,725 | 99,9 | 2,25 | 0 |
| 60 min | | 99,9 | | 99,9 |
| 5 min | 0,686 | 99,9 | 1,125 | 0 |
| 30 min | | 99,9 | | 0 |

### Example 6. Study of antiviral activity against Herpes Simplex Virus type-1 (HSV-1), in vitro.

The studies against the RSV virus were conducted with active ingredients A2 and A3. The cells used in the tests were human laryngeal carcinoma cells (ATCC CCL-23) (HEp-2), and the Respiratory Syncytial Virus (RSV, Long strain, negative-sense single-stranded RNA virus, enveloped).

The methodology used in the tests was carried out following the described steps: previously, the cells were cultured in 96-well microplates with Dulbecco's modified Eagle's medium (DMEM) supplemented with 10% fetal bovine serum, 100 µg/mL streptomycin, 100 IU/mL penicillin, and 2.5 µg/mL amphotericin B. The cells were maintained at 37°C and 5% CO2 for 24 hours until the formation of a monolayer (10^4 cells/mL).

The virucidal assay for HSV-1 demonstrated that the active ingredient A2 inactivated the viral particle (99.9%) in the shortest contact time (5 minutes), down to the lowest concentration tested (0.686%). For active ingredient A3, 99.9% inactivation was achieved only at the highest concentration (2.25%), with the contact time with the viral particle increased to 60 minutes (Table 5).

The results presented are extended to Herpesvirus type 1* (HSV-1, KOS strain, double-stranded DNA virus, enveloped), where HSV-1 belongs to the Alphaherpesvirinae subfamily, Simplexvirus genus, Herpesviridae family. The Alphaherpesvirinae subfamily includes, in addition to HSV-1, Herpesviruses types 2 (HSV-2) and 3 (HHV-3 or Varicella-Zoster).

**Table 5. Viral Inactivation Percentage (% IV, from 0 to 99.9%) by Virucidal Effect for HSV-1, at Different Contact Times and Concentrations of Actives A2 and A3.**

| | HSV-1 | | HSV-1 | |
|---|---|---|---|---|
| | Active A2 | | Active A3 | |
| time | Concentration (%) | % IV | Concentration (%) | % IV |
| 5 min | 1,725 | 99,9 | 2,25 | 0 |
| 60 min | | 99,9 | | 99,9 |
| 5 min | | 99,9 | | 0 |
| 30 min | 0,686 | 99,9 | 1,125 | 0 |

The virucidal assay for Herpes Simplex Virus type 1 (HSV-1), strain AR, which is resistant to Acyclovir, of a topical spray formulation containing the active compound LS3V2 at a 1% concentration demonstrated that the active compound inactivates HSV-1 (Acyclovir-resistant) after 5 minutes of contact, in vitro. The test formulation (topical spray containing 1% LS3V2) inactivated 99.9% of the viral particles (HSV-1, strain AR - Acyclovir-resistant) at the shortest contact time of 5 minutes. The base formulation did not inhibit viral infection, showing results similar to the viral control (VC).

The virucidal assay for Herpes Simplex Virus type 1 (HSV-1), strain AR, resistant to Acyclovir, of a topical gel formulation for genital use containing the active compound L3 at a 0.75% concentration demonstrated that the active compound inactivates HSV-1 (Acyclovir-resistant) after 30 minutes of contact, in vitro. The test formulation (topical gel for genital use containing 0.75% L3) inactivated 99.9% of the viral particles (HSV-1, strain AR - Acyclovir-resistant) at the evaluated contact time of 30 minutes. The base formulation did not inhibit viral infection, showing results similar to the viral control (VC).

The virucidal assay for Herpes Simplex Virus type 1 (HSV-1), strain AR, resistant to Acyclovir, of a topical gel formulation for labial use containing the active compound L3 at a 0.75% concentration demonstrated that the active compound inactivates HSV-1 (Acyclovir-resistant) after 30 minutes of contact, in vitro. The test formulation (topical gel for labial use containing 0.75% L3) inactivated 99.9% of the viral particles (HSV-1, strain AR - Acyclovir-resistant) at the evaluated contact time of 30 minutes. The base formulation did not inhibit viral infection, showing results similar to the viral control (VC).

**Table 6. Antiviral Activity Results for Formulations Containing Active Compound L3 Against Acyclovir-Resistant Herpes Simplex Virus Type 1 (HSV-1 AR), in vitro.**

| | **Topical Spray 1%** | **Genital gel** | **Labial gel** |
|---|---|---|---|
| **time** | **Result** | **Result** | **Result** |
| 5 min | Inactivated | Not tested | Not tested |
| 30 min | Not tested | Inactivated | Inactivated |

The results presented for the assays of these three formulations are for Herpes Simplex Virus type 1, strain AR - Acyclovir-resistant (HSV-1 AR), belonging to the Alphaherpesvirinae subfamily, Simplexvirus genus, Herpesviridae family. The Alphaherpesvirinae subfamily includes, in addition to HSV-1, Herpes Simplex Viruses types 2 (HSV-2) and 3 (HHV-3 or Varicella-Zoster). The assays were conducted in a NB-2 laboratory (Biosafety Level 2), following "Good Laboratory Practices" (GLP) and in accordance with ANVISA Recommendations (Art. 1 and Art. 3 of IN 04/13 and IN 12/16).

The methodology for the virucidal test of all formulations in spray, genital gel, and labial gel was as follows: the virus (HSV-1 strain AR) was added and kept in contact for pre-established times as per Table 6. After this period, the virus was inoculated into cell cultures (Vero ATCC CCL-81) and incubated for 72 hours at 37°C and 5% CO2. The viral inactivation capacity of the formulation was determined using the TCID50 assay (REED; MÜNCH, 1938). The following controls were used under the same experimental conditions: i) Virus treated only with cell culture medium - Viral Control (VC); ii) Virus treated with base formulation, in the absence of the active compound L3SV2 - Base Control; and iii) Culture medium in the absence of virus - Cell Control (CC).

## Claims

1. **ANTIVIRAL COMPOSITION, characterized by** comprising encapsulating agents, whether free or not, said agents being cyclic oligosaccharides, at least one antiseptic active agent in an inclusion compound with a cyclic oligosaccharide, and excipients or a mixture thereof.

2. **ANTIVIRAL COMPOSITION,** according to claim 1, **characterized in that** the encapsulating agents are beta-cyclodextrin and/or its structural variants, or a mixture thereof.

3. **ANTIVIRAL COMPOSITION,** according to claims 1 and 2, **characterized in that** the structural variants of beta-cyclodextrin are selected from the group consisting of hydroxypropyl-beta-cyclodextrin, betadex sulfobutyl ether sodium, hydroxymethyl-beta-cyclodextrin, methyl-beta-cyclodextrin, 2,6-dimethyl-beta-cyclodextrin, and hydroxyethyl-beta-cyclodextrin.

4. **ANTIVIRAL COMPOSITION,** according to claim 1, **characterized in that** the antiseptic agent may be cationic, anionic, zwitterionic, or neutral, and selected from the bisbiguanide class, such as chlorhexidine or its acetate, gluconate, or digluconate salts; or from the tetracycline class, such as doxycycline, eravacycline, minocycline, omadacycline, or combinations thereof.

5. **ANTIVIRAL COMPOSITION,** according to claims 1 and 4, **characterized in that** the antiseptic agent may be selected from the group comprising classes of saturated or unsaturated organic acids with a main carbon chain containing between 2 and 8 carbon atoms, or combinations thereof, in concentrations ranging from 0.01 to 30% w/v.

6. **ANTIVIRAL COMPOSITION,** according to claims 1, 4, and 5, **characterized in that** the antiseptic agent is selected from the group comprising classes of essential oils or hydrosols of essential oils derived from natural sources such as thyme, eucalyptus, oregano, basil, peppermint, rosemary, wild rosemary, pequi, geranium, citronella, palmarosa, pitanga, cypress, melaleuca, ginger, lemongrass, lemon grass, or propolis, or combinations thereof, in concentrations ranging from 0.05 to 99% w/w.

7. **ANTIVIRAL COMPOSITION,** according to claim 1, **characterized in that** the excipients are selected from the group consisting of humectants, emulsifiers, preservatives, thickeners, sweeteners, stabilizers, colorants, antioxidants, surfactants, and/or flavoring agents, in concentrations ranging from 0.02 to 99% w/w.

8. **ANTIVIRAL COMPOSITION,** according to claims 1 to 7, **characterized in that** the antiseptic agent and the encapsulating agent are present in molar ratios of 8:1 to 1:10, respectively.

9. **ANTIVIRAL COMPOSITION,** according to claims 1 to 8, **characterized by** comprising 0.01 to 20% w/w of the free encapsulating agent in excess in the product and/or its structural variations or combinations thereof.

10. **USE OF THE ANTIVIRAL COMPOSITION,** as defined in any of claims 1 to 9, **characterized by** containing beta-cyclodextrin and/or its structural variations or combinations thereof, as a modulator of the antiviral activity of antiseptic agents.

11. **USE OF THE ANTIVIRAL COMPOSITION,** as defined in any of claims 1 to 10, **characterized by** being active against viruses selected from the groups of the orders Herpesvirales, Caudovirales, Mononegavirales, Nidovirales, Picornavirales, Tymovirales, and the family Retroviridae

12. **USE OF THE ANTIVIRAL COMPOSITION,** according to claim 11, **characterized by** being preferentially active against the virus from the *Herpesviridae* family and the *Retroviridae* family.

13. **USE OF THE ANTIVIRAL COMPOSITION,** according to claims 11 and 12, **characterized by** being for pharmacological application, such as antibiotics, tablets or injectables, and related formulations.

14. **USE OF THE ANTIVIRAL COMPOSITION,** according to claims 11 to 13, **characterized by** being for veterinary application.

15. **USE OF THE ANTIVIRAL COMPOSITION,** according to claims 11 to 14, **characterized by** being for the topical and systemic treatment of diseases caused by HSV-1, HSV-2, varicella-zoster (HHV-3), Epstein-Barr (HHV-4), cytomegalovirus (HHV-5), HHV-6, HHV-7, and Kaposi's sarcoma (HHV-8).

16. **USE OF THE ANTIVIRAL COMPOSITION,** according to claims 11 to 15, **characterized by** the topical treatment being selected from the group comprising epithelial tissues, skin, mouth, fingers, nails, hair, mammary glands, perineal region, genitals, or mucous membranes.

17. **USE OF THE ANTIVIRAL COMPOSITION,** as defined in any one of claims 1 to 10, **characterized by** being a sanitizing agent for surface disinfection, for the production of formulations for topical use or for environmental treatment, surface cleaning, air purification, or in air purification equipment.

18. **USE OF THE ANTIVIRAL COMPOSITION,** according to claim 17, **characterized by** being in aqueous solution, powder, paste, wet gel, xerogels, aerosols, spray, foam, tablets, capsules, or in adhesives, inserted or not into cellulosic materials, polymers, or fabrics.
